Europäisches Patentamt

**European Patent Office**

**Office européen des brevets**

(19)

(11) Publication number:

**0 305 194**
**A2**

# (12) EUROPEAN PATENT APPLICATION

(21) Application number: **88307919.6**

(22) Date of filing: **26.08.88**

(51) Int. Cl.⁴: **A 61 K 45/08**
A 61 K 31/40, A 61 K 9/00

(30) Priority: **28.08.87 AU 4034/87**

(43) Date of publication of application:
**01.03.89 Bulletin 89/09**

(84) Designated Contracting States:
**AT BE CH DE ES FR GB GR IT LI LU NL SE**

(71) Applicant: **Regulin Limited**
**Level 12 222 Kingsway**
**South Melbourne Victoria (AU)**

(72) Inventor: **Staples, Linton Drew**
**103 Normanby Road**
**East Kew, 3102, Victoria (AU)**

(74) Representative: **Field, Roger Norton et al**
**Brewer & Son Quality House 5-9, Quality Court Chancery Lane**
**London WC2A 1HT (GB)**

(54) Veterinary composition.

(57) A veterinary composition comprising an effective amount of

(I) a primary biologically active agent selected from melatonin or related chemicals of the indole class or mixtures thereof;

(II) at least one complementary biological active agent; eg oestrus synchronizing agents; pheromones; or substances involved with brain function, including neurotransmitters; and

(III) a veterinarily acceptable carrier or excipient.

EP 0 305 194 A2

## Description

## VETERINARY COMPOSITION

This invention relates to a method of regulating the reproductive functions of animals, particularly domesticated ruminants, and to veterinary compositions for use in such a method.

In our earlier Australian Patent Application No. 78305/81 there is described a method of artificially mimicking changing photoperiod, thereby changing the breeding activity of sheep, goats, deer and other seasonally breeding animals, by the judicious feeding of Melatonin or similarly effective related chemicals. In subsequent applications 38111/85 and 72915/87, (European Patent Publications Nos 0169856 and 0246910) we describe improved methods of administering melatonin to achieve this same effect.

The role of seasonal environmental factors, in particular the photoperiod, in determining the breeding period of sheep is well established. Under natural conditions the changing of day length such as when summer leads to autumn is the main trigger to the reproductive system to commence ovarian cyclicity. Our previous applications show that melatonin treatment can mimic the effects of short day length on ewes, such that the breeding season is advanced and basal prolactin levels are depressed.

However whilst these prior art methods have proved to be effective, more extensive modification of breeding activity and/or other physiological effects, would be most desirable.

It is accordingly an object of the present invention to overcome, or at least alleviate, one or more of the difficulties related to the prior art.

Accordingly, in a first aspect, there is provided a veterinary composition including an effective amount of

(i) a primary biologically active agent selected from melatonin or related chemicals of the indole class, as hereinafter defined, or mixtures thereof,

(ii) at least one complementary biologically active agent,

(iii) a veterinarily acceptable carrier or excipient, The veterinary composition may function to regulate seasonal breeding activity and/or seasonal physiological responses of an animal to be treated.

We have discovered that other biologically active agents in conjunction with melatonin may have further beneficial effects, beyond the effect of either active alone.

The primary biologically active agent of the veterinary composition according to this aspect of the present invention may be selected from Melatonin or related or derived chemicals of the indole class. While the present invention describes the use of Melatonin per se, it has been established in the prior art that certain other related or derived molecules may exert a similar biological effect. Thus Kennaway et al. (1986) have demonstrated that certain classes of Melatonin derivative or analogue (notably those halogenated at the indol-6 position, those carrying a saturated bond at the indol-2,3 position, and those arising from cleavage of the indolyl ring) have a similar, although lesser, effect on serum prolactin levels when administered to sheep, compared to Melatonin. (Kennaway, D.J., P. Royles, E.A. Dunstan and H.M. Hugel, 1986. Prolactin Response in Border Leicester x Merino Ewes to Administration of Melatonin, Melatonin Analogues, a Melatonin Metabolite and 6-Methoxybenzoxazolinone. Australian Journal of Biological Sciences, 39:427-433). Similarly, Frohn et al (1980) using a biological assay highly specific for Melatonin-like activity, have established the biological activity of a variety of analogues, derivatives, and related indole compounds, relative to Melatonin itself. (Frohn, M.A., C.J. Seaborn, D.W. Johnson, G. Phillipou, R.F. Seamark and C.D. Matthews, 1980. Structure-activity Relationship of Melatonin Analogues. Life Sciences, 27:2043-2046). Accordingly, the active agent of the coated veterinary implant according to this aspect of the present invention may be selected from

MELATONIN (N-acetyl 5-methoxy tryptamine)

MELATONIN analogues substituted at the amino nitrogen, e.g.:

N-propionyl/N-butyryl

MELATONIN analogues substituted at the 5- position,

e.g.: 5-ethoxy (i.e. N-acetyl O-ethyl serotonin)

5-propoxy (i.e. N-acetyl O-propyl serotonin)

MELATONIN analogues substituted at the 6- position,

e.g.: Halo-substituted: 6-fluoro/6-bromo/6-chloro/6-iodo

Hydroxy- substituted

Suphatoxy- substituted

Alkyl- substituted: 6-methyl/6-ethyl/6-isopropyl etc.

Alkoxy- substituted, e.g. 6-methoxy

Silylated, e.g. 6-(t-butyl)dimethylsiloxy

SATURATED MELATONIN DERIVATIVES, such as (2,3-dihydro)melatonin and 6-chloro-(2,3-dihydro)melatonin (together with all the 5-, 6- and N- substituted saturated derivatives thereof as described above)

RING-CLEAVED MELATONIN derivatives (including all the 5- 6- and N- substituted derivatives thereof as described above, and similarly, saturated derivatives thereof). such as

N-acetyl 5-hydroxy kynurenamine

N-acetyl 5-methoxy kynurenamine

RELATED INDOL (SEROTONIN) derivatives, such as

N-acetyl serotonin

N,O-diacetyl serotonin

N-acetyl tryptamine
Serotonin
5-methoxy tryptophol
5-methoxy tryptamine
OTHER DERIVATIVES and RELATIVES of MELATONIN, such as
N-alkyl and/or N,N-dialkyl analogues of tryptamine, serotonin and melatonin
N-aryl and/or N,N-diaryl analogues of tryptamine, serotonin and melatonin
Similarly, mixed N-alkyl, N-aryl derivatives 5-substituted derivatives of tryptamine, Ureido- analogues of tryptamine and serotonin; or mixtures thereof.

By "seasonal physiological responses" as used herein in the description and claims we mean changes in the physiology of the animal to be treated which are affected, under natural conditions, by season. Such aspects include changes to the growth and colour or quality of pelage, and development of horn or antler, appetite, reproductive activity and the like.

The amount of melatonin or related chemicals of the indole class as defined above in the formulation may be sufficient to provide for an elevation of the circulating blood concentration of melatonin or equivalent active material to greater than the normal daytime level in an untreated animal for a period of up to several weeks.

It is recognised that melatonin analogues may show a relationship between administered dose, blood level and biological effect which is different from that seen with melatonin itself. Thus the primary biologically active agent may contribute approximately 2.5 to 98% by weight based on the total weight of the veterinary composition, depending on the species and breed to be treated, the type of primary active, the type of excipient and the secondary active or actives included in the composition.

For animals such as sheep, deer and the like the active agent may be present in amounts of from approximately 75 to 98% by weight based on the total weight of the veterinary composition.

For animals such as mink and fox amounts of active agent of from approximately 25 to 75% by weight may be used.

The at least one complementary biologically active agent of the veterinary composition according to this aspect of the present invention may be selected from any one or more of the following classes
    (A) oestrus synchronizing agents;
    (B) other agents which directly or indirectly increase ovarian function;
    (C) pheromones or pheromone analogues;
    (D) substances involved with thyroid function; and
    (E) substances involved with brain function, including neurotransmitters;
or mixtures thereof.

Examples of agents falling within each class are as follows:

Class A
- Progesterone or other progestagens which may be used to control the timing of oestrus and/or to permit the expression of oestrus at the first ovulation of the season or induced season.
- Other steriods in general - natural or synthetic.
- Prostaglandins - particularly those of the E and F class, and particularly Prostaglandin PGF2 and its analogues in all their forms.

Class B
- Conjugated steriods which are themselves antigenic or which are antigenic in combination with adjuvants or enhancers of immune responses - e.g.: andostenedione conjugate (Fecundin), oestrone conjugate, oestradiol-17-beta conjugates or mixtures thereof.
- Gonadotrophin or mixtures or preparations with gonadotropin activity whether homologous or heterologous to the species in which the veterinary composition is to be used.
Examples of such gonadotrophic substances include:

- Pregnant Mare Serum Gonadotrophin (PMSG)
    -Follicle Stimulating Hormone (FSH) including, but not exclusively those of equine, ovine, bovine and porcine origin. - Luteinizing hormone and its biologically active analogues.
- Inhibin, whether of testicular or ovarian origin, and whether homologous or heterologous, or whether obtained by genetic engineering, or by purification from inhibin-rich, naturally-occurring tissues or fluids.
- Immunizing forms of Inhibin such as chemically altered Inhibin, heterologous Inhibin, or Inhibin conjugated with other substances.
- Releasing factors in general, and in particular those associated with the control of gonadotrophin secretion, such as Gonadotropin Releasing Hormone (GnRH) also known as Luteinizing Hormone Releasing Hormone (LHRH) or its natural or synthetic analogues including those of agonistic or antogonistic action.
- Prolactin or prolactin analogues, or substances other than the primary active which affect prolactin levels (e.g. bromocriptine)

Class C
- Olfactory stimuli which are known to affect the activity of the hypothalamo-pituitary axis (phermones), whether natural or synthetic e.g.: those produced by goat bucks, deer stags, boars and rams during and aroung the natural or induced breeding season.

Class D
- Thyroid hormones, such as T4, T3 and Thyrotropins, whether natural or synthetic.

CLass E
- Neutotransmitter substances.
More preferably the at least one complementary biologically active agent is selected from progestagens, prostaglandins, conjugated steroids, gonadotrophins or mixtures thereof.

The at least one complementary biologically active agent may be administered in combination with the primary biologically active agent. Alternatively the secondary biologically active agent may be adminstered before or after the administration of the primary agent.

The veterinarily acceptable carrier or excipient may include non-toxic oils of vegetable or mineral origin, polymers, alcohols and other diluents. Useful non-toxic carriers may include peanut oil, hydrogenated vegetable oil, glycols, ethanol, isopropanol, polyols such as glycerol, sorbitol and propylene glycol and ketones such as acetone. The veterinarily acceptable carrier or excipient may include a compressible pharmaceutical vehicle selected to control the release rate of the active agent. The carrier or excipient may be present in amounts of approximately 1 to 95% by weight based on the total weight of the veterinary composition.

Such a vehicle may be a high molecular weight form of ethyl cellulose. The form of ethyl cellulose may be chosen from the range of ethoxylated cellulose derivatives sold under the trade designation "Ethocel" and available from the Dow Chemical Company. Ethocel Standard and Ethocel Medium, both of viscosity grades between 7 cP and 100 cP, are preferred.

Alternatively, or in addition, the veterinary carrier or excipient may include an acid salt. The acid salt may be a phosphate salt. The vehicle may be an alkaline earth metal salt. A calcium phosphate is preferred. A hydrated acid salt may be used. A dibasic calcium phosphate dihydrate is preferred. The acid salt may be a calcium phosphate of the type sold under the trade designation "Encompress" and available from Edward Mendell Co. Inc., New York, United States of America.

Other compounding agent may also be included. Such agents may include colouring agents, flavourant, fillers, binders, surfactants and the like.

The veterinary composition may preferably take the form of a veterinary implant. Such implants have been described in European Patent Publications Nos 0169856 and 0246910, the entire disclosure of each of which is incorporated herein by reference. These implants comprise melatonin, a related indole or derivative thereof as the active ingredient and additionally a veterinally acceptable carrier or excipient. They may have a coating which is formed from a physiologically compatible polymeric coating composition.

The secondary biologically active agent may be incorporated in the veterinary implant, or may be administered separately.

The implants may be individually or severally loaded into a separate chamber of a plastic cartridge. The plastic cartridges may be placed into a "gun" and the implant delivered subcutaneously through a large bore needle.

Although subcutaneous implantation is our preferred route for administering these implants, they may be effective when administered orally, vaginally, intraperitoneally (I.P.), percutaneously or intramuscularly (I.M.). The IM or I.P routes may be used in particular in fur-bearing animals, such as mink and angora rabbits.

In a further aspect of the present invention there is provided a method of regulating the seasonal breeding activity and/or related physiological responses in animals which method includes administering to an animal to be treated an effective amount of

(I) a primary biologically active agent selected from melatonin or related chemicals in the indole class, as hereinbefore defined, or mixtures therof; and

(II) at least one complementary biologically active agent.

Preferably the at least one complementary biologically active agent is selected from any one of the following classes:

(A) oestrus synchronizing agents;

(B) other agents which directly or indirectly increase ovarian function;

(C) pheromones or pheromone analogues;

(D) substances involved with thyroid function; and

(E) substances involved with brain function, including neurotransmitters.

More preferably the at least one complementary biologically active agent is selected from progestagens, prostaglandins, conjugated steriods, Gonadotrophins or mixtures thereof.

In a preferred form there is provided a method of regulating the seasonal breeding activity and/or related physiological responses of animals which method includes administering to an animal to be treated an effective amount of a veterinary composition including an effective amount

(I) a primary biologically active agent selected from melatonin or related chemicals of the indole class, as

4

hereinbefore defined, or mixtures thereof,

(II) at least one complementary biologically active agent,

(III) a veterinarily acceptable carrier or excipient. The veterinary composition may function to regulate seasonal breeding activity and/or seasonal physiological responses of an animal to be treated.

In a still further aspect of the present invention there is provided a kit of parts including

(i) a primary biologically active agent selected from melatonin or related chemicals of the indole class, as hereinbefore defined, or mixtures thereof, in a suitable container or delivery vehicle, and

(ii) at least one complementary biologically active agent in a suitable container or delivery vehicle.

The primary biologically active agent may be provided in the form of a veterinary implant as described above.

The at least one complementary biologically active agent may be selected from any one of the following classes;

(A) oestrus synchronizing agents;

(B) other agents which directly or indirectly increase ovarian function;

(C) pheromones or pheromone analogues;

(D) substances involved with thyroid function; and

(E) substances involved with brain function, including neurotransmitters.

More preferably, the at least one complementary biologically active agent is selected from progestagens, prostaglandins, conjugated steroids, Gonadotrophins or mixtures thereof. The at least one complementary biologically active ingredient may also be supplied in the same or a different implant. The at least one complementary biologically active agent may be in an injectable form, or in the form of an intrauterine or intravaginal device, for example a sponge.

Where progesterone is used this may be provided in a progesterone-releasing controlled intravaginal delayed release device CIDR.

In the following description, reference will be made to the administration of the veterinary composition to sheep. It should be understood, however, that such animals are mentioned for illustrative purposes only and the veterinary composition is applicable to animals generally, including sheep, goats, horses, deer, pigs, ferrets, mink, fox. The veterinary composition may further be applied to the regulation of seasonal breeding or other physiological events in birds, such as turkeys, geese, and fowls and in fish including sturgeon, trout, salmon and eels where applications may include alterations to the time of spawning or of the smolt process.

The method of regulating the reproductive functions in animals may be such as to advance or delay the onset of oestrus or breeding activity, to induce or delay the onset of ovulation or sperm production, or to increase the rate of ovulation or sperm production. In advancing or delaying the onset of ovulation or sperm production, the duration of the animal's breeding season may be altered. Some or all of these effects may be obtained in just the season of treatment or may continue for an extensive period, e.g. two to four years, or throughout the reproductive life of the animal.

The animal to be treated may be a mature animal. The seasonal breeding activity may be modified by accelerating the onset of oestrus or the breeding cycle.

The animal to be treated may be a pre-pubescent animal and the seasonal breeding activity is modified by delaying the onset of puberty and thus the onset of the breeding season is altered over a number of years.

In general, the animal to be treated will be a female. However, alternatively or in addition the male of the species may be treated. This is preferably for deer and goats and to a lesser extent, sheep. Where changes in pelage (fur/wool/coat/pelt production etc.) are sought, the composition may be administered to both entire and castrate male and female animals.

In a further preferred aspect of this invention, the reproductive functions of female animals may be regulated by increasing the rate of ovulation in such treated animals.

In a further related aspect, this invention provides a method of regulating the physiological characteristics of animals which method includes inserting a coated veterinary implant of the type described above into an animal to be treated.

With respect to sheep, for example, the induction of an earlier onset to the breeding season has a number of advantages including allowing earlier joining for lamb drop in more favourable weather, longer growth periods for lambs and minimizing potential labour clashes for management of lambing and for marking with that required for other enterprises on the farm such as harvesting the crops.

Melatonin treatment permits a defined joining time since treated ewes reliably respond to "ram effect" and therefore mate and concieve at about 3 to 4 weeks after introduction of rams. Although melatonin implants are typically used in sheep in conjunction with the "ram effect" they will induce changes in reproductive activity without the additional use of rams.

Melatonin treatment may shorten the conception period so that lambing period will be condensed (this allows for an even crop of lambs and maximization of labour inputs over a short lambing period).

Melatonin treatment induces the normal peak seasonal ovulation rates to occur early in the season. Fecundity (number of lambs/ewe joined) is therefore increased by 14 to 30%. This is due mostly to an increased occurrence of twins or triplets and to a decrease in the proportion which are not pregnant.

Certain other physical characteristics of animals may be regulated in a similar way by means of the coated sustained-release melatonin implant described herein. The physiological characteristics which may be regulated are those usually associated with reproductive activity or seasonal changes, and include changes in the thickness or the colour of the animal's coat, pelt or fur, and the development of horn or antler.

We have now discovered unexpectedly that supplementary treatment with melatonin as described herein may enhance the effect of various other treatments designed to manipulate various aspects of the reproductive and/or physiological responses of target animals. Thus, in practice, compositions or treatments which combine melatonin with one or more secondary biologically active agents have been found to have greater, or more beneficial, activity than compositions or treatments which involve either agent alone.

We have found that combination of melatonin treatment with oestrus synchronising treatment (progesterone priming) of ewes leads to a significant shortening of the mean time to conception, compared to treatment with progesterone alone (see example 1). A corresponding phenomenon has been observed in deer (example 3). Similarly, supplementary melatonin treatment has been found to overcome some of the limitations of steroid immunization, in particular preventing the delay in mating pattern seen with immunized ewes, and reducing the occurrence of barren ewes, typical of steroid immunized animals (see example 2). In addition, supplementary melatonin treatment has been found to further enhance the combined effect of progestagen oestrus synchronization and gonadotrophic stimulation on conception rates in artificial insemination (example 4).

The present invention will now be more fully described with reference to the accompanying examples. It should be understood, however, that the examples are illustrative only and should not be taken in any way as a restriction on the generality of the invention described above.


EXAMPLES


Example 1 - Field Trial 1: Class A combined treatment:


The use of Melatonin in conjunction with progesterone priming.

The effect of Regulin® melatonin implants and type C CIDR's (progesterone-releasing intravaginal devices) on the early breeding of 84 Booroola Merino X perendale (B x P) and 142 Waihorn Romney (R) ewes were compared in four treatment groups:
Group (1) melatonin plus CIDR;
Group (2) melatonine only
Group (3) CIDR only;
Group (4) control.

In this example treatment groups 1 and 2 were implanted with melatonin on 12th December and 23rd December respectively. Melatonin on 12th December and 23rd December respectively. Melatonin treated ewes were grazed separately from untreated ewes. Treatment groups 1 and 3 had CIDR's inserted to each ewe on 7th January and removed on 19th January after a period of at least 5 weeks isolation from rams. Rams were fitted with a harness containing a wax marking crayon which marked ewes on the day of mating. The ewes were joined in four mating groups, Romney ewes were mated to Booroola Merino (FF genotype) x Romney rams in melatonin treated and untreated groups, and Booroola x Perendale ewes were mated to Booroola (+ + genotype) x Romney rams in treated and untreated groups.

The results are summarized in Table 1.

**Table 1:** Lambing results

| Group | Treatment | Ewes lambing/ ewes present | | Ewes lambing multiple/ewe lambing % | | Lambs born/ ewes mated | |
|---|---|---|---|---|---|---|---|
| | | BxR | R | BxR | R | BxR | R |
| 1 | Melatonin CIDR | 67 | 76 | 57 | 24 | 1.14 | 0.94 |
| 2 | Melatonin only | 84 | 81 | 19 | 32 | 1.00 | 1.06 |
| 3 | CIDR only | 62 | 74 | 23 | 8 | 0.73 | 0.79 |
| 4 | Control | 75 | 57 | 7 | 6 | 0.80 | 0.56 |

The mean interval from ram introduction to first observed oestrus in the B x P ewes was 8, 25, 16 and 26 days respectively for the four treatments, and in the Romney ewes the interval was 16, 26, 27 and 30 for groups 1, 2, 3 and 4 respectively.

These results indicate a synergistic effect of the combined treatments on the onset of first oestrus in the Merino x Romney breed. Similarly there was a synergistic effect of the combined treatment in % of ewes lambing multiples in B x P ewes (Table 1). Synergistic effect in lambing percentages was also seen in ewes of the Boorolla x Romney and Romney breeds when progesterone intravaginal possaries and melatonin implants were used together. In the BxR ewes the combined treatment resulted in at least 0.41 additional lambs per ewe treated compared with just the progesterone alone, and in the Romney ewes this increase was 0.15 additional lambs in the combined treatment compared with the use of progesterone alone.

Example 2 - Field Trial 2: Class B combined treatment:

The effect of melatonin in conjunction with steroid immunization on the reproductive performance of ewes joined early in the season.

Breed: Mature Border Leicester x Merino ewes mated to Dorset rams

Joining Date: 18th December, 1987, about three months prior to the natural Autumn seasonal peak in reproductive performance.

Location: Kybybolite, South Australia

Independent Supervisor: Eugene Dunstan - Sheep industry specialist of the South Australia Government Department of Agriculture.

The treatment groups were as summarised in the following table:

| | Treatment compared | Ewes observed |
|---|---|---|
| Compared: | Group 1 - Control | n = 81 |
| | Group 2 - Regulin[R] melatonin implant | n = 78 |
| | Group 3 - Recundin[R] (androstendione immunization) | n = 81 |
| | Group 4 - Regulin + Fecundin | n = 83 |

The results are summarized in Table 2. While the combined treateament in this particular example had only a marginal effect on the delay in mating pattern, it had a significant improving effect on the conception rate, as well as reducing the number of barren ewes.

Example 3 - Field Trial 3

There are significant advantages to be gained by advancing the calving season of red deer hinds from early summer to spring, particularly that of improved pasture utilisation.

The aim of this trial was to evaluate the efficacy of melatonin feeding and melatonin implants to advance seasonal reproductive activity.

From 10th December until 6th March, at 1500 h each day, 30 hinds were fed 5 mg/head/day melatonin incorporated in a ration of deer nuts (MF). During the same period 16 hinds were each given two subcutaneous constant melatonin releasing implants (Genelink, Australia) replaced at approximately 28 day intervals (MI). A further six hinds remained untreated (C).

Ovulation was synchronised in MF and MI groups by either intravaginal progesterone (CIDR) only (inserted 17th February and removed 4th March) or by two prostaglandin F2 (PG) injections (250 ug closprostenol, Estrumate, ICI, on 21st February and 3rd March) and in the control group by CIDR only.

Evidence of a previous ovulation, determined by laparoscopy on 12th March, was recorded in 9/20 MF, 12/16 MI and 0/6 C hinds. Nine of the MF hinds which did not ovulate however, did not consistently eat the treated ration. of the MF hinds which ate their ration, 4/5 CIDR and 5/6 PG treated had a curpus luteum. Of the MI hinds 7/8 CIDR and 5/8 PG treated hinds had a corpus luteum.

TABLE 2

## KYBYBOLITE, SOUTH AUSTRALIA - BLxM
### Joined 18.01.87

| GROUP | n | FETUSES 0 | 1 | 2 | 3 | FETUSES/ CONC. RATE | % MATING EWE PREGNANT | 0 | 1 | 2 | 3 | MEAN MATING DAY | MEAN CONC. DAY |
|---|---|---|---|---|---|---|---|---|---|---|---|---|---|
| CONTROL | 81 | 4 | 33 | 41 | 3 | $1.530^{A}$ | $1.610^{X}$ | 1 | 80 | 9 | – | 20.48 | 21.54 |
| % | | 4.94 | 40.7 | 50.6 | 3.7 | | | 1.27 | 98.8 | 11.1 | – | | |
| REGULIN | 78 | 4 | 20 | 52 | 2 | $1.666^{AB}$ | $1.756^{XX}$ | 2 | 76 | 6 | 1 | 19.82 | 20.12 |
| % | | 5.1 | 25.6 | 66.7 | 2.56 | | | 2.56 | 97.4 | 7.7 | 1.28 | | |
| FECUNDIN | 81 | 13 | 19 | 40 | 9 | $1.555^{B}$ | $1.852^{X}$ | 5 | 76 | 11 | – | 24.52 | 25.89 |
| % | | 16.1 | 23.5 | 49.4 | 11.1 | | | 6.2 | 93.8 | 13.6 | – | | |
| REGULIN & FECUNDIN | 83 | 3 | 21 | 37 | 21 | $1.952^{C}$ | $2.025^{X}$ | 2 | 81 | 13 | 1 | 23.03 | 25.70 |
| | | 3.6 | 25.3 | 44.5 | 25.3 | | | | 2.4 | 97.6 | 15.7 | 1.2 | |

VALUES WITH DIFFERENT SUPERSCRIPTS DIFFER SIGNIFICANTLY, $P < 0.05$

This trial has demonstrated that both daily melatonin feeding and melatonin implants are capable of advancing the breeding season of yearling red deer hinds, with both methods appearing reliable once the problem of feed acceptance is overcome. The results from prostaglandin treatment suggests that those animals were cycling prior to the time of synchronisation, and that the breeding season could be advanced still further using the melatoniin treatments described here.

Example 4 - Field Trial 4: Class A plus B multiple combined treatment.

The effect of melatonin treatment on AI conception rates following progestagen oestrus synchronisation and gonadotrophic stimulation.
Breed: Milk flock of Assaf breed (Awasi x E, Friesian)
Location: Lower Gallilee, Israel.
Indepentent Supervisors: Dr. Reznekov (Sheep Insemination Centre), Nitzan Ziv (Alon Chemicals) and Haim Leibowitz (Charman, Fertility Committee, Ministry of Agriculture).
The overall treatment protocol was as follows:

```
Day  0    10-4-88    Implant with Regulin^R melatonin implant
Day 28    07-5-88    Insert progesterone sponges intravaginally
Day 38    17-5-88    Introduce teaser rams
Day 41    20-5-88    Remove sponges, inject PM5G (0600 h)
Day 43    22-5-88    Inseminate at 50 and 60 h after PMSG
                     injection
Day 57    06-6-88    Introduction of rams with markers to look
                     for "returns", continued to Day 64
Day 92    11-7-88    Ultrasound pregnancy check
```

The treatment groups and results are summarised in the Table 3.

Table 3: Results of Field Trial 4 - Israeli Assaf Sheep:

| | CONCEPTION RATE (%) | | |
| --- | --- | --- | --- |
| AI at months post-partum | Control | + Melatonin | Diff(%) |
| 2 | 58 | 86 | +29 |
| 3 | 60 | 97 | +37 |
| 4 | 87 | 95 | +8 |
| "Trouble"* ewes | 45 | 67 | +22 |

* These ewes had failed to conceive at an earlier AI attempt.

In all treatment groups, supplementary treatment with melatonin led to significant improvement in conception rate.
In each of the foregoing examples, a variety of agents were used in attempts to improve the reproductive behaviour or efficiency of animals. The mechanisms by which progesterone priming and PMSG treatment exert their effect are well known. Melatonin acts not on the reproductive system directly but on photoperiod perception. Accordingly, it is surprising that supplementary treatment with melatonin-like agents would have any beneficial effect on the actions of the other agents considered here. More surprisingly, we have discovered a greater, synergistic effect, in our experiments as described here.

Finally, it is to be understood that various other modifications and/or alterations may be made without departing from the spirit of the present invention as outlined herein.

## Claims

1. A veterinary composition comprising an effective amount of
(I) a primary biologically active agent selected from melatonin or related chemicals of the indole class as hereinbefore defined, or mixtures thereof;
(II) at least one complementary biological active agent; and,
(III) a veterinarily acceptable carrier or excipient.

2. A veterinary composition according to claim 1 wherein the primary biologically active agent is present in amounts of from approximately 2.5 - 98% by weight based on the total weight of the veterinary composition.

3. A veterinary composition according to either of claims 1 and 2 wherein the at least one complementary biologically active agent is selected from any one of the following classes;
(A) oestrus synchronizing agents;
(B) other agents which directly or indirectly increase ovarian function;
(C) pheromones or pheromone analogues;
(D) substances involved with brain function, including neurotransmitters.

4. A veterinary composition according to claim 3 wherein the at least complementary biological active agent is selected from progestagens, prostaglandins, conjugated steroids, Gonadotrophins or mixtures thereof.

5. A veterinary composition according to any one of the preceding claims wherein the veterinary acceptable carrier or excipient is selected from non-toxic oils of vegetable of mineral origin, polymers, alcohols or other diluents.

6. The veterinary composition according to any one of the preceding claims wherein the veterinary acceptable carrier or excipient further includes approximately 1-95% by weight, based on the total weight of the veterinary composition, of a compressible pharmaceutical vehicle.

7. A veterinary composition according to any one of the preceding claims wherein the veterinary composition is in the form of a veterinary implant.

8. A method of regulating the seasonal breeding activity and/or related physiological responses in animals which method includes administering to an animal to be treated an effective amount of
(I) a primary biologically active agent selected from melatonin or related chemicals in the indole class, as hereinbefore defined, or mixtures thereof; and
(II) at least one complementary biologically active agent.

9. A method according to claim 8 wherein the at least one complementary biologically active agent is selected from any one of the following classes;
(A) oestrus synchronizing agents;
(B) other agents which directly or indirectly increase ovarian function;
(C) pheromones or pheromone analogues;
(D) substances involved with thyroid function; and
(E) substances involved with brain function, including neurotransmitters.

10. A method according to claim 9 wherein the at least one complementary biological active agent is selected from progestagens, prostaglandins, conjugated steroids, Gonadotrophins or mixtures thereof.

11. A method of regulating the seasonal breeding activity and/or related physiological responses in animals which method includes administering to an animal to be treated an effective amount of a veterinary composition according to any one of claims 1 to 7.

12. A kit of parts including
(I) a primary biologically active agent selected from melatonin or related chemicals of the indole class, hereinbefore defined, or mixtures thereof, in a suitable container of delivery vehicles; and
(II) at least one complementary biologically active agent in a suitable container or delivery vehicle.

13. A kit of parts according to claim 12 wherein the primary biologically active agent is provided in the form of a veterinary implant.

14. A kit of parts according to either of claims 12 and 13, wherein the at least one complementary biologically active agent is selected from any one of the following classes;
(A) oestrus synchronizing agents;
(B) other agents which directly or indirectly increase ovarian function;
(C) pheromones or pheromone analogues;
(D) substances involved with thyroid function; and
(E) substances involved with brain function, including neurotransmitters.

15. A kit of parts according to claim 14, wherein the at least one complementary active agent is selected from progestagens, prostaglandins, conjugated steroids, Gonadotrophins or mixtures thereof.

16. A kit of parts according to claim 15, wherein the at least one complementary biologically active agent is provided in an injectable form, in the form of intraruterin or intravaginal device or a combination thereof.